# EUROPÄISCHE PATENTANMELDUNG

(11) **EP 1 880 713 A1**
(43) Veröffentlichungstag der Anmeldung: **23.01.2008**
(21) Anmeldenummer: 07013853.2
(22) Anmeldetag: 14.07.2007
(51) Int. Cl.: A61K 8/92, A61K 33/08, A61P 17/02

(54) **Verwendung einer Zubereitung aus Calciumhydroxid und Öl zur Hautwundversorgung**

(30) Priorität: 15.07.2006 DE 102006032887
(71) Anmelder: MetaCura FZE, Dubai (AE)
(72) Erfinder: Ollert, Karl E.S., 81825 München (DE); Tapparo, Ottaviano, Dr.Dr.h.c.Prof., 81669 München (DE)
(74) Vertreter: Blasi, Ralph

(57) **Zusammenfassung**

Die vorliegende Erfindung bezieht sich auf die Verwendung einer Zubereitung aus Calciumhydroxid, einem Öl vegetarischen, animalischen oder mineralischen Ursprungs, oder aus den Substanzen solcher Öle gewonnenen synthetischen Ölen zur Hautwundversorgung, Förderung der Hautneubildung oder Hautregeneration, wobei das Calciumhydroxid in dem Öl als Trägersubstanz gebunden ist.

## Beschreibung

Die Erfindung betrifft die Verwendung einer Zubereitung aus Calciumhydroxid und einem Öl vegetarischen, animalischen, oder mineralischen Ursprungs, oder aus den Substanzen solcher Öle gewonnenen synthetischen Ölen zur Hautwundversorgung, Förderung der Hautneubildung oder Hautregeneration.

Schnitt-, Schürf- oder Stichwunden entstehen im Alltag häufig durch Stöße und Stürze, oder durch Unfälle mit spitzen bzw. scharfen Gegenständen. Der erste Schritt bei der Wundversorgung ist immer das Säubern und Desinfizieren. Dann folgt die Wundbehandlung bzw. der Schutz der Wunde durch Verbandstoffe. Bei stark blutenden Wunden steht die Blutstillung im Vordergrund.

Typische Anzeichen einer infizierten Wunde sind:
• Schmerzen, teilweise pulsierend oder klopfend
• Schwellung
• Hautrötung
• Druckempfindlichkeit
• Temperaturerhöhung

Neben Eiterbakterien sind vor allem auch die Tetanuserreger gefährlich. Schnittwunden, die mehr als einen Zentimeter lang sind, müssen i.d.R. beim Arzt primär versorgt d.h. desinfiziert, genäht oder geklammert werden. Stichwunden sind sofort zu desinfizieren. Verletzungen, bei denen Fremdkörper in die Haut eindringen, sind besonders infektionsgefährdet.

Meistens bildet sich bei blutenden Wunden innerhalb von wenigen Minuten ein Wundschorf. Der Wundschorf schützt die Wunde gegen das Eindringen von Bakterien, weitere Blutverluste und das Austrocknen. Mit zunehmendem Heilungsprozess fällt er von alleine ab.

Zur Unterstützung der Wundheilung sind folgende Mittel bekannt:

### 1) Mittel mit desinfizierender Wirkung

Jod ist ein wirksames Mittel gegen Bakterien, Pilze und Viren. Die entsprechenden Desinfektionsmittel töten die Keime ab. Sie können auf kleine und große Wunden, sowie auf das umliegende Hautgewebe direkt aufgetragen werden. Jod-Tinktur enthält reines Jod und wird nur noch selten eingesetzt. Heute verwendet man überwiegend Povidon-Jod (PVP-Jod). Bei diesem Wirkstoff ist Jod so eingebunden, dass es nur langsam freigesetzt wird. Dadurch sind diese Desinfektionsmittel besser verträglich. PVP-Jod wird nicht nur zur Desinfektion von Wunden und Schleimhäuten, sondern auch zur Operationsvorbereitung oder zur antiseptischen Händewaschung eingesetzt. Das Mittel ist schnell wirksam. Untersuchungen zu Folge soll es die Keimzahl in einer Wunde innerhalb von einer Minute von 1 Million Keime auf höchstens 10 Keime reduzieren. Jodhaltige Medikamente eignen sich nicht zur Anwendung bei bestehender Überfunktion der Schilddrüse, da die Tätigkeit der Schilddrüse durch Jod angeregt werden kann. Jodhaltige Medikamente können bei gleichzeitiger Anwendung von silberhaltigen Desinfektionsmitteln oder enzymhaltigen Wundbehandlungsmitteln deren Wirkung herabsetzen. Es kann auch in Verbindung mit Wasserstoffperoxid (Desinfiziens) oder Taurolidin (Chemotherapeutikum) zu Wechselwirkungen kommen.

Verschiedene Alkohole wie Ethanol, Isopropanol oder Phenoxyethanol wirken ebenfalls sehr schnell und haben ein breites Wirkspektrum. Auch bei diesen Produkten werden Bakterien, Viren und Pilze abgetötet. Voraussetzung für die Wirksamkeit ist eine ausreichend hohe Konzentration des Alkohols. Manche Mittel enthalten zusätzlich weitere antiseptisch wirksame Stoffe. Reiner Ethanol oder Isopropanol wird heute vor allem zur Hautdesinfektion vor Injektionen oder zur Händedesinfektion eingesetzt. Diese Mittel sollen nicht auf Schleimhäute oder in die Nähe der Augen geraten. Beim Auftragen auf offene Hautstellen entsteht ein unangenehmes Brennen. Phenoxyethanol dagegen brennt nicht bei Kontakt mit Wundflächen. Die Kombination Phenoxyethanol/Octenidin wird zur Desinfektion von Schleimhäuten und Wunden empfohlen. Bei gleichzeitiger Anwendung von Antiseptika mit dem Wirkstoff PVP-Jod kann es zu braunen und violetten Verfärbungen kommen.

Chlorhexidin wird zur Behandlung oberflächlicher Wunden eingesetzt. Zur Wunddesinfektion kommt der Wirkstoff als Salbe, Spray oder Puder zum Einsatz. Chlorhexidin darf nicht in die Nähe der Augen oder auf Schleimhäute geraten. Puder darf nicht auf tiefe oder nässende Wunden sowie Geschwüre aufgetragen werden, da die dadurch entstehende Kruste die Vermehrung von Bakterien begünstigt.

Tyrothricin wird als Gel oder Puder zur Wundbehandlung bei Schnitt-, Brand- und Schürfwunden eingesetzt. Die Zubereitungen können vorbeugend oder auch bei bestehenden Infektionen, beispielsweise bei eitrigen Entzündungen, angewandt werden.

Benzethonium wirkt desinfizierend bei kleineren Wunden oder Verbrennungen. Auch dieser Wirkstoff kann sowohl zur Behandlung bakterieller Hautinfektionen als auch zur Vorbeugung verwendet werden. Keine Anwendung im Auge, keine Behandlung großflächiger Wunden.

Ethacridinlactat wirkt gegen Entzündungen, die durch äußere Verletzungen entstanden sind. Der Wirkstoff dringt schnell in das Gewebe ein. Ethacridinlactat wird als Salbe, aber auch als Lösung angeboten. Dadurch werden desinfizierende Umschläge oder Teilbäder möglich. Ethacridinhaltige Zubereitungen sind gelb gefärbt.

### 2) Mittel zur Wundheilung

Wundsalben beschleunigen den Heilungsprozess. Sie sollten jedoch nur auf oberflächliche Wunden aufgetragen werden, die nicht bluten oder nässen. Wenn sich die Haut nach oberflächlichen oder tiefen Verletzungen rötet, ist das ein Zeichen dafür, dass das Immunsystem auf den äußeren Reiz reagiert. In betroffenen Arealen zirkulieren verstärkt Zytokine wie Tumornekrosefaktor (TNF) oder Interleukin-1 (IL-1.). Die Botenstoffe führen unter anderem zu einer Gefäßerweiterung und die Haut wird besser durchblutet. TNF und IL-1 fördern indirekt aber auch die Wundheilung. So wiesen Wissenschaftler nach, dass die Zytokine auch die Produktion von neuen Keratinozyten ankurbeln, und so defektes Gewebe wieder repariert werden kann. Die Entzündung ist für die Wundheilung essenziell, sie darf aber nicht zu lange anhalten. Strömen zu viele Entzündungsmediatoren in die Wunde, wird der Heilungsprozess aufgehalten. Deshalb gilt es, die Entzündung rechtzeitig mit antiinflammatorischen Dermatika zu bremsen. Die im Handel erhältlichen Dexpanthenol-Salben beschleunigen die Wundheilung und auch die Hautrötung klingt schneller ab. Die Substanz wird zunächst in der Haut zu Panthothensäure umgewandelt. Diese nutzt der Organismus unter anderem zur Produktion von Coenzym A, das dann unter anderem in die für die Barriereschicht notwendigen Ceramide eingebaut wird. und

Auch Zinkoxid fördert die Wundheilung. Die Zubereitungen können mehrmals am Tag aufgetragen werden. Zinkoxid wird manchmal mit Lebertran kombiniert. Lebertran verstärkt die hautpflegenden Effekte.

Bei den pflanzlichen Pflegemitteln hat sich der Einsatz von Extrakten des Purpursonnenhuts (Echinacea purpurea), der Zaubernuss (Hamamelis virginiana), der Kamille (Matricaria chamomilla) und der Ringelblume (Calendula offcinalis) bewährt. Die Arzneipflanzen wirken antientzündlich und unterstützen die wundheilenden Prozesse.

### 3) Wundverbände

Die heutigen Wundschnellverbände sind mittlerweile so atmungsaktiv, dass sie dem Heilungsprozess nicht im Wege stehen. Sie schützen die Haut vor weiteren Verunreinigungen. Größere Wundflächen können mit sterilen Kompressen abgedeckt werden, wobei die Kompressen dann mit einem elastischen Mullverband fixiert werden.

Eine Aufgabe der Erfindung ist es, eine pharmazeutische Zubereitung zur Hautwundversorgung, Förderung der Hautneubildung oder Hautregeneration bereitzustellen, die gleichzeitig zur Desinfektion der Wunde, zur Förderung der Wundheilung und zumindestens in begrenztem Rahmen als Abdeckung der Wunde dient.

Eine weitere Aufgabe der Erfindung ist es, eine pharmazeutischen Zubereitung zur Hautwundversorgung, Förderung der Hautneubildung oder Hautregeneration bereitzustellen, die auch bei tiefen, blutigen Wunden eingesetzt werden kann, die desinfizierend wirkt, ohne die Gefahr einer nekrotischen Wirkung auf das Gewebe zu haben.

Eine weitere Aufgabe der Erfindung ist es, eine pharmazeutischen Zubereitung zur Hautwundversorgung, Förderung der Hautneubildung oder Hautregeneration bereitzustellen, die gleichzeitig zumindest partiell als Wundabdeckung bzw. Wundpflaster dienen kann.

Die vorstehenden Aufgaben werden durch die Verwendung einer pharmazeutische Zubereitung aus Calciumhydroxid, einem Öl vegetarischen, animalischen oder mineralischen Ursprungs, oder aus den Substanzen solcher Öle gewonnenen synthetischen Ölen erreicht. Während Calciumhydroxid eine desinfizierende Wirkung hat, übernimmt das Öl die Funktion eines Wundverschlusses. In der Kombination beider Substanzen ist zudem eine deutliche Wundheilfunktion nachweisbar. Die Bindung des Calciumhydroxids in die Öl -Trägersubstanz verhindert auch eine nekrotische Wirkung auf die Wunde.

Es kommt also bei der erfindungsgemäßen Mischung darauf an, dass das Calciumhydroxid durch die ölige Trägerszubstanz gebunden Wird und an der Wundwand erst wirksam werden kann, wenn es aus dem Öl herausdiffundiert ist.

Würde man das Öl durch Wasser ersetzen, würde die erfindungsgemäße Bindung des Calciumhydroxids an die ölige Trägersubstanz nicht eintreten und das Calciumhydroxid würde in der erfindungsgemäßen Konzentration zu einer Nekrotisierung des Hautgewebes führen. Nur eine ölige Trägersubstanz ist stabil genug, um das Calciumhydroxid auch in hohen Konzentrationen langsam und gleichmäßig an die Wundwand abgeben zu können und so die erwünschte therapeutische Wirkung, so wie sie in der Patentbeschreibung beschrieben ist abgeben zu können.

Die erfindungsgemäße pharmazeutische Zusammensetzung besitzt die Funktion eines Desinfektionsmittels, einer Wundsalbe und einer Wundabdeckung bei Hautdefekten. Ohne einer Theorie anzuhängen, wird angenommen, dass dies auf folgendem beruht: Die ölige Substanz mit Calciumhydroxid bildet eine geschlossene Schicht mit Membranwirkung: Innen hält sie die neu gebildeten Stammzellen zusammen, stimuliert diese, und außen tötet sie Bakterien ab. Der bakteriostatische Effekt der erfindungsgemäßen Mischung ist gerade nicht so stark, als dass auch die für die Hautneubildung, bzw. -regeneration maßgeblichen Stammzellen abgetötet werden würden.

Wird die Paste innerhalb kurzer Zeit nach der Verletzung in der blutigen Wunde aufgetragen, verhindert sie, dass in der Wunde eine Entzündung eine Entzündung entsteht und sich eine Nekroseschicht bildet. Das Gewebe zieht sich daher nicht zurück sondern dessen Regenerationsprozess beginnt ohne Verzögerung innerhalb kurzer Zeit nach der Verletzung.

Die erfindungsgemäße Mischung ist daher den Desinfektionsmitteln und Wundsalben, die die Bildung einer Nekroseschicht unterstützen deutlich überlegen.

### Bevorzugte Ausführungsformen

Die pharmazeutische Zusammensetzung umfasst Calciumhydroxid und ein Öl vegetarischen, animalischen oder mineralischen Ursprungs, oder ein aus den Substanzen solcher Öle gewonnenes synthetisches Öl. Der im folgenden verwendete Ausdruck "Zubereitung" bezeichnet eine pharmazeutische Zubereitung (im folgenden manchmal auch als Gemisch bezeichnet), die mindestens die oben genannten Bestandteile enthält. Sie eignet sich insbesondere zur Verabreichung an Menschen oder Tieren zur Hautwundversorgung, Hautneubildung, bzw. Hautregeneration.

Wenn in der pharmazeutischen Zubereitung ein vegetarisches Öl verwendet wird, kann dieses aus der Gruppe ausgewählt sein, die aus Kaktus-, Soja-, Sonnenblumen-, Rüb-, Baumwollsaat-, Lein-, Rizinus-, Palm-, Palmkern-, Kokos- und Olivenoelen besteht. Vorzugsweise werden Sonnenblumen-, Kaktus-, Soja-, Olivenöle verwendet. Diese Öle sind besonders stabil.

Das Öl kann ferner ein fettes tierisches Öl sein, das aus der Gruppe ausgewählt ist, die aus Fischölen, Klauenölen, Fettölen und Talgen besteht.

Vorzugsweise wird ein Klauenöl, z.B. Oleum Pedum, oder Schweinefettöl verwendet. Diese Öle sind besonders stabil.

Wenn ein mineralisches Öl verwendet wird, kann dieses Steinöl sein.

Die pharmazeutische Zubereitung kann durch ein physikalisches Verfahren verdickt werden. Ein derartiges physikalisches Verfahren kann darin bestehen, dass mindestens ein Inhaltsstoff der pharmazeutischen Zubereitung durch Aufschäumen verdickt wird.

Die pharmazeutische Zubereitung kann ferner einen pharmazeutisch verträglichen Hilfsstoff enthalten.

Der pharmazeutisch verträgliche Hilfsstoff kann ein Verdickungsmittel sein.

Der pharmazeutisch verträgliche Hilfsstoff kann ein zwei- oder mehrwertiger Alkohol sein, vorzugsweise Glycerin sein.

Der erfindungsgemäßen Zubereitung kann ein Vitalstoff zugesetzt werden. Vorzugsweise wird der Vitalstoff aus der Gruppe ausgewählt ist, die aus Vitaminen, Spurenelementen, Fettsäuren, sekundären Pflanzenstoffen und Antioxidantien besteht.

Vorzugsweise werden die Vitamine aus der Gruppe ausgewählt sind, die aus Q 10, Vitamin E, Vitamin F, Vitamin A, und Vitamin C besteht. Insbesondere bevorzugt wird Vitamin C maisfrei verwendet.

Als Spurenelement kann Zink zugesetzt werden.

Als Fettsäuren können Omega 3 und 6 Fettsäuren zugesetzt werden.

Als sekundäre Pflanzenstoffe können oligomere Proanthocyanidine (OPC) oder Resveratrol verwendet werden.

Ferner kann die pharmazeutische Zubereitung kolloidales Silber enthalten. Hierdurch wird der bakteriostatische Effekt verstärkt.

Die erfindungsgemäße Zubereitung kann Salicylsäure oder Harnstoff enthalten. Diese Stoffe wirken als Trägerstoffe und ermöglichen ein besseres Eindringen der Wirksubstanz Calciumhydroxid in die Haut. Vorzugsweise werden diese Substanzen einer erfindungsgemäßen Zubereitung zugesetzt, mit der unblutige Hautwunden behandelt werden sollen.

Die erfindungsgemäße Zubereitung kann ferner Kollagen oder Hyaluronsäure enthalten. Diese Substanzen unterstützen die Wundheilung.

Zur Herstellung der erfindungsgemäßen Zubereitung wird das Calciumhydroxid gewöhnlich in Mengen von 1-90 Gew.-%, vorzugsweise 5-80 Gew.-% bezogen auf das Gesamtgewicht der Zubereitung aus Calciumhydroxid und Öl zugegeben.

Die erfindungsgemäße Zubereitung kann in der Konsistenz einer Creme, vorzugsweise mit weniger als 30 Gew.-% Calciumhydroxid hergestellt werden.

Alternativ kann die Zubereitung als knetbare Zubereitung, vorzugsweise mit mehr als 50% Calciumhydroxid hergestellt werden.

Bei offenen Wunden wird vorzugsweise eine pharmazeutische Zusammensetzung verwendet, die 30 Gew.-% oder weniger Calciumhydroxid enthält.

Bei Wunden, die bereits eine erste Hautschicht oder Wundschorf gebildet haben, wird vorzugsweise eine pharmazeutische Zubereitung mit einem Calciumhydroxidgehalt von mehr als 30 Gew.-%, insbesondere bevorzugt mehr als 50 Gew.-%, verwendet. Durch die größere Konzentration an Calciumhydroxid wird trotz der Hautschicht oder des Wundschorfs, die eine natürliche Barriere bilden, eine ausreichendes Diffundieren des Calciumhydroxids ermöglicht, so dass dieses seine Wirkung, wie vorstehend beschrieben, in der Wunde entfalten kann.

Durch den erhöhten Anteil Calciumhydroxid wird die Konsistenz der Paste stabiler. Sie eignet sich hervorragend zum Auftragen auf Hautpartien. Das Calciumhydroxid auf der Haut schützt die Haut vor Infektionen und desinfiziert diese. Darüberhinaus regt sie offenbar die Bildung neuer Hautzellen an, was die Beobachtungen belegen. Zu diesem Zweck dring vermutlich das Calciumhydroxid (in seiner erhöhten Konzentration) unter die Hautschicht ein und fördert die Teilung und Ausdifferenzierung der Stammzellen, was zu einer deutlichen Verdichtung der Hautzellen führt.

Das Öl kann der erfindungsgemäßen Zubereitung in Mengen von 10-99 Gew.-%, vorzugsweise 20-90 Gew.-%, jeweils bezogen auf das Gesamtgewicht der erfindungsgemäßen Zubereitung zugegeben werden.

Wenn das erfindungsgemäße Gemisch eine besonders geschmeidige und glatte Konsistenz aufweisen soll, kann ihm auch noch Vaseline einverleibt werden. Gewöhnlich kann Vaseline in Mengen von 1-60 Gew.-%, zweckmäßigerweise in Mengen von 10-60 Gew.-%, vorzugsweise in Mengen von 20-50 Gew.-%, jeweils bezogen auf das Gesamtgewicht der Zubereitung zugesetzt werden.

Die Applikation des erfindungsgemäßen Gemischs auf oder in das Hauttrauma kann - je nach seiner Konsistenz mittels Spateln oder Pinseln erfolgen.

Für das erfindungsgemäße Gemisch gibt es zahlreiche Einsatzmöglichkeiten in der Dermatologie da das erfindungsgemäße Gemisch auf Hauttraumen applizierbar ist und am jeweiligen Applikationsort sofort eine verbesserte und beschleunigte Hautwundheilung auslöst.

Die erfindungsgemäße Zubereitung kann zur Hautregeneration eingesetzt werden, insbesondere zur Hautregeneration bei von Cellulitis oder Akne betroffenen Hautpartien.

Die erfindungsgemäße Zubereitung kann ferner zur Hautwundversorgung durch Desinfektion und zur Entzündungsreduzierung verwendet werden.

Schließlich ist es auch denkbar, die in der Einleitung genannten desinfizierenden und die Wundheilung unterstützenden Substanzen, inbesondere Jod bzw. Povidon-Jod (PVD-Jod), Phenoxyethanol, Chlorhexidin, Tyrothricin, Phenoxyethanol, Chlorhexidin, Benzethonium, Ethacridinlactat, Dexpanthenol-Salben, Extrakten des Purpursonnenhuts (Echinacea purpurea), der Zaubernuss (Hamamelis virginiana), der Kamille (Matricaria chamomilla) und der Ringelblume (Calendula officinalis), in Kombination mit der erfindungsgemäßen Zubereitung zu verwenden.

### Fallbeschreibungen

Von 2005 bis Mitte 2006 wurde die erfindungsgemäße Mischung in 45 Fällen angewendet. Bei allen Anwendungen hat die erfindungsgemäße Mischung eine beschleunigte und reizlosere Wundheilung , im Vergleich zur Wundheilung ohne Hilfsmittel, ergeben. In Tabellen 1 und 2 sind Anwendungsfälle zusammengestellt, die die Wirkung der erfindungsgemäßen Mischung deutlich machen.

Es wurden folgende Mischungen verwendet:
Mischung 1: 8 Gew.-% Ca(OH)₂, 46 Gew.-% Öl, 46 Gew.-% Vaseline
Mischung 2: 14 Gew.-% Ca(OH)₂, 436 Gew.-% Öl, 43 Gew.-% Vaseline
Mischung 3: 22 Gew.-% Ca(OH)₂, 39 Gew.-% Öl, 39 Gew.-% Vaseline
Mischung 4: 40 Gew.-% Ca(OH)₂, 30 Gew.-% Öl, 30 Gew.-% Vaseline
Mischung 5: 34 Gew.-% Ca(OH)₂, 33 Gew.-% Öl, 33 Gew.-% Vaseline
Mischung 6: 50 Gew.-% Ca(OH)₂, 25 Gew.-% Öl, 25 Gew.-% Vaseline

Die Mischungen wurden jeweils wie folgt hergestellt:

Oleum Pedum wird in einem Topf mit Mischer vorgelegt und die Vaseline homogen eingearbeitet. Anschließend wird das gesiebte Calciumhydroxid in kleinen Portionen zugegeben und zu einer homogenen Paste verarbeitet. Anschließend wird dispergiert.

**Tabelle 1: Anwendungsfälle der erfindungsgemäßen Mischungen 1-3**

| Art der Wunde | Zeitraum der Beobachtung | Beschreibung der Wunde | Beschreibung der Handhabung | Ergebnis |
|---|---|---|---|---|
| Offen, blutend | | | | |
| Kratzer | 03.11.2005 - 01.12.2005 | 1 mm tief, 2 mm breit, blutend, 1,5 cm lang, an der Hand | Direktes Aufbringen der Mischung 1 auf die Kratzwunde nach Säuberung der Wunde mit Wasser; Abdeckung mit einem Hansa-Plast-Heftpflaster; anschließend Tetanusprophylaxe tägliches Wechseln des Pflasters; keine erneute Reinigung der Wunde | Reiz und komplikationsloser Heilungsverlauf, nach 1 Woche war die Wunde zu einem kleinen Strich reduziert, nach 3 Wochen war der Kratzer fast vollständig verheilt |
| Kratzer | 25.12.2005 - 09.01.2006 | 1 mm tief, 1 mm breit, blutend, 1 cm lang, am Finger | Direktes Aufbringen der Mischung 1 auf die Kratzwunde nach Säuberung der Wunde mit Wasser; Abdeckung mit einem Hansa-Plast-Heftpflaster; anschließend Tetanusprophylaxe tägliches Wechseln des Pflasters; keine erneute Reinigung der Wunde | Reiz und komplikationsloser Heilungsverlauf, nach 1 Woche war die Wunde zu einem kleinen Strich reduziert, nach 3 Wochen war der Kratzer fast vollständig verheilt |
| Großflächige Hautwunde | 07.03.2006 - 23.04.2006 | Vollständige Abschürfung der Haut 1 mm tief, blutend , ca. 10 x 5 cm, an der Vorderseite des rechten Unterschenkels, | Direktes Aufbringen der Mischung 2 auf die Wundfläche nach Säuberung mit Wasser, Abdeckung mit einem nicht saugfähigen, nicht haftenden Pflaster; anschließend Tetanusprophylaxe täglicher Wechsel des Pflasters und erneutes Auftragen der Mischung ohne erneute Reinigung der Wunde; | Keine Schwellung, keine Schmerzen, , ganz leichte Sekretbildung, Bildung einer dünnen Fibrinschicht, reiz und komplikationsloser, völlig unauffälliger Wundheilungsprozess Wundfläche war nach 14 Tagen mit junger, roter Haut ersetzt; |
| Großflächige Hautwunde im Vergleich | 07.03.2006 - 23.04.2006 | vergleichbare Scheuerwunde an der linken Vorderseite des Unterschenkels | Übliche Wundversorgung mit einem nicht saugfähigen / nicht haftenden Heftpflaster, tägliche Reinigung der Wunde mit Wasser und Auswechseln des Verbandes | Sehr starke Sekretbildung, mehrmalige leichte Entzündungszeichen wie Schwellung, deutliche Schmerzen, mehrmaliges Verkleben der neu gebildeten Fibrinschicht an den Verband, Bildung einer dicken Kruste, die erst nach 6 Wochen vollständig durch junge rote Haut ersetzt wurde. |
| 5 Fälle von eitrigen Pickeln | 05.04.2006 - 02.07.2006 | Entzündete eitrige Pickel am Oberschenkel, nach dem Ausdrücken blutig | Unmittelbar nach dem Ausdrücken mit der Mischung 3 jeweils an der Wundseite großzügig aufgetragen und mit einem nicht haftenden Heftpflaster abgedeckt | Keine erneute Entzündung nach dem Ausdrücken und einmaliger Behandlung nach 10 Tagen vollständig verheilt, fast nicht mehr sichtbar. |
| 3 Fälle von eitrigen Pickeln im Vergleich | 05.04.2005 - 02.07.2005 | 3 entzündete eitrige Pickel am Oberschenkel, nach dem Ausdrücken blutig | Nach dem Ausdrücken lediglich Reinigung mit Wasser | Bei 2 Pickeln erneute Entzündung ; Entzündungsrückgang und Verschluss der Hautoberfläche nach jeweils 5 Tagen, alle drei Pickel nach 4 Wochen noch sichtbar |

Die Erfinder haben auch Versuche gemacht, bei denen die cremige Paste auf Wunden aufgetragen wurde, die oberflächig geschossen waren und nicht mehr bluteten. Diese wiesen eine dünne, rote Hautschicht auf. Es wurde keine Wirkung festgestellt. Die Hautschicht hat verhindert, dass sich das Calciumhydroxid aus dem Öl gelöst hat und eine Wirkung entfalten konnte. Deshalb wurde der Anteil des Calciumhydroxids deutlich gesteigert und die folgenden Fallbeschreibungen (Tabelle 2) dokumentiert.

**Tabelle 2: Anwendungsfälle der erfindungsgemäßen Mischungen 4-6**

| Nichtblutende Wunden nach der Bildung der ersten Schicht | | | | |
|---|---|---|---|---|
| Schürfwunden | 24.04.2005 - 01.07.2005 | 1 Schürfwunde (2 x 2 cm), unterhalb des Knies, bereits mit einer jungen, roten Hautschicht überzogen | Es wurde Mischung 4 großzügig aufgetragen; keine weitere Abdeckung, da die Haftung sehr gut ist; tägliches Auftragen der Paste | Nach vier Wochen Behandlung, verglichen mit der Kontrollseite, nahm die Rotfärbung deutlich ab. Bei leichtem Druck auf die Wunde hatte die Haut dieselbe Farbe wie die Umgebung und war somit als Wunde nicht mehr erkennbar. |
| Schürfwunde zum Vergleich | 24.04.2005 - 01.07.2005 | 1 Schürfwunde (2 x 2 cm), unterhalb des Knies, bereits mit einer jungen, roten Hautschicht überzogen | Keine Behandlung | Nach vier Wochen immer noch deutliche Rotfärbung der Wunde, die auch auf leichtem Druck die Farbe nicht änderte. |
| 2 entzündete Pickel | 05.04.2005 - 10.07.2005 | 2 entzündete Pickel, | Unmittelbar nach dem Ausdrücken mit der Mischung 5 auf den Pickel großzügig aufgetragen, kein Heftpflaster | Keine erneute Entzündung, nach dem Ausdrücken und einmaliger Behandlung nach 10 Tagen vollständig verheilt, fast nicht mehr sichtbar. |
| 2 blutige, entzündete Pickel zum Vergleich | 05.04.2005 - 10.07.2005 | 2 entzündete Pickel, in der Nähe der mit der Paste behandelten Pickel | Nach dem Ausdrücken lediglich Reinigung mit Wasser | Keine erneute Entzündung; aber beide nach 10 Tagen noch deutlich sichtbar, auch nach 4 Wochen immer noch sichtbar |
| Schnittwunde Wundränder adaptiert | 13.06.2006 - 10.07.2006 | 1,5 mm tief, blutig, 1 cm lang, Fuß | Direktes Aufbringen der Mischung 6 auf die Schnittwunde nach einmaliger Reinigung der Wunde mit Wasser; anschließend Tetanusprophylaxe tägliches Auswechseln der Paste, keine erneute Reinigung der Wunde | Reiz und komplikationsloser Heilungsprozess, nach 5 Tagen war noch ein unauffälliger Strich zu sehen, nach 2 Wochen war der Schnitt praktisch nicht mehr zu sehen |
| Schnittwunde Wundränder adaptiert | 16.06.2006 - 10.07.2006 | 2 mm tief, blutig 0,7 cm lang, Hand | Direktes Aufbringen der Mischung 6 auf die Schnittwunde nach einmaliger Reinigung der Wunde mit Wasser; anschließend Tetanusprophylaxe tägliches Auswechseln der Paste, keine erneute Reinigung der Wunde | Reiz und komplikationsloser Heilungsprozess, nach 5 Tagen war noch ein unauffälliger Strich zu sehen, nach 2 Wochen war der Schnitt praktisch nicht mehr zu sehen |

Hieraus lässt sich der Schluss ziehen, dass mit der erfindungsgemäßen Substanz auch nach der Bildung einer ersten dünnen Hautschicht, die Regeneration der Haut gefördert werden kann, wenn der Anteil des Calciumhydroxids erhöht wird. Gleiches gilt bei einer Schnittwunde, bei der die Wundränder adaptiert sind.

## Patentansprüche

1. Verwendung einer Zubereitung aus Calciumhydroxid, einem Öl vegetarischen, animalischen oder mineralischen Ursprungs, oder aus den Substanzen solcher Öle gewonnenen synthetischen Ölen zur Hautwundversorgung, Förderung der Hautneubildung oder Hautregeneration, wobei das Calciumhydroxid in dem Öl als Trägersubstanz gebunden ist.

2. Verwendung nach Anspruch 1, wobei das Öl vegetarischen Ursprungs aus der Gruppe ausgewählt ist, die aus Sonnenblumen-, Kaktus-, Soja-, Olivenölen besteht.

3. Verwendung nach Anspruch 1, wobei das Öl tierischen Ursprungs ein Klauenöl ist.

4. Verwendung nach mindestens einem der vorhergehenden Ansprüche, wobei mindestens ein Inhaltsstoff der pharmazeutischen Zubereitung durch physikalische Behandlung verdickt ist.

5. Verwendung nach mindestens einem der vorhergehenden Ansprüche, wobei die Zubereitung ferner ein Verdickungsmittel enthält.

6. Verwendung nach mindestens einem der vorhergehenden Ansprüche, wobei die Zubereitung ferner Glycerin enthält.

7. Verwendung nach mindestens einem der vorhergehenden Ansprüche, wobei die Zubereitung ferner Vaseline enthält.

8. Verwendung nach mindestens einem der vorhergehenden Ansprüche, wobei die Zubereitung ferner einen Vitalstoff enthält, der aus der Gruppe ausgewählt ist, die aus Vitaminen, Fettsäuren, Spurenelementen, sekundären Pflanzenstoffen und Antioxidantien besteht.

9. Verwendung nach mindestens einem der vorhergehenden Ansprüche, wobei die pharmazeutische Zubereitung ferner kolloidales Silber enthält.

10. Verwendung nach mindestens einem der vorhergehenden Ansprüche, wobei die pharmazeutische Zusammensetzung 30 Gew.-% oder weniger Calciumhydroxid enthält.

11. Verwendung nach mindestens einem der vorhergehenden Ansprüche, wobei die pharmazeutische Zusammensetzung 50 Gew.-% oder mehr Calciumhydroxid enthält.

12. Verwendung nach mindestens einem der vorhergehenden Ansprüche, wobei die pharmazeutische Zusammensetzung ferner Salicylsäure oder Harnstoff enthält.

13. Verwendung nach mindestens einem der vorhergehenden Ansprüche, wobei die pharmazeutische Zusammensetzung ferner Kollagen oder Hyaluronsäure enthält.

14. Verwendung nach mindestens einem der vorhergehenden Ansprüche, wobei die Hautregeneration sich auf kosmetische Anwendungen bezieht.

15. Verwendung nach mindestens einem der vorhergehenden Ansprüche, wobei die Hautregeneration sich auf Behandlungen zur Hautstraffung bezieht.

16. Verwendung nach mindestens einem der vorhergehenden Ansprüche, wobei die Hautregeneration sich auf von Cellulitis oder Akne betroffener Hautpartien bezieht.

17. Verwendung nach mindestens einem der vorhergehenden Ansprüche, wobei die Hautwundversorgung durch Desinfektion und zur Entzündungsreduzierung durchgeführt wird.
